# FASCICULE DE BREVET EUROPEEN

(11) **EP 1 515 688 B1**
(45) Date de publication et mention de la délivrance du brevet: **24.07.2013**
(21) Numéro de dépôt: 03757134.6
(22) Date de dépôt: 10.06.2003
(51) Int. Cl.: A61K 8/58, A61K 8/34, A61K 8/49, A61K 8/73, A61K 8/44, A61K 8/35, A61K 8/37, A61Q 5/00, A61Q 19/00, A61K 8/19, A61K 8/41, A61K 8/63, A61K 8/67, A61Q 5/02, A61Q 5/06, A61K 8/64

(54) **Utilisation d'un complexe salen-manganèse comme agent protecteur des mélanocytes du follicule pileux et applications cosmétiques**
Verwendung vom Salen-Mangan-Komplexen als Schutzmittel für Melanocyten in Haarfollikeln und kosmetische Verwendungen
Use of salen-manganese complexes as protective agents for hair follicule melanocytes and cosmetic uses thereof

(30) Priorité: 11.06.2002 FR 0207137; 19.06.2002 US 389708 P
(43) Date de publication de la demande: 23.03.2005
(73) Titulaire: L'Oréal, 75008 Paris (FR)
(72) Inventeur: COMMO, Stéphane, F-75015 Paris (FR); GAILLARD, Olivier, F-91470 Forges-les-Bains (FR); BERNARD, Bruno, 92400 Courbevoie (FR)
(74) Mandataire: Allab, Myriam
(86) Numéro de dépôt international: PCT/FR2003/001729
(87) Numéro de publication internationale: WO 2003/103616

(56) Documents cités:
- EP-A- 0 327 345
- EP-A- 0 545 147
- EP-A- 0 580 409
- WO-A-99/39728
- ES-A- 2 052 450
- US-A- 5 965 157
- DATABASE WPI Week 199511 Derwent Publications Ltd., London, GB; AN 1995-077960 XP002231404 & JP 07 002677 A (TOYO SEITO KK) 6 janvier 1995 (1995-01-06)
- DATABASE WPI Week 199939 Derwent Publications Ltd., London, GB; AN 1992-189207 XP002231405 & JP 04 124122 A (SHISEIDO CO. LTD.) 24 avril 1992 (1992-04-24)

## Description

La présente invention se rapporte à l'utilisation cosmétique d'un agent mimétique de l'activité de la DOPAchrome tautomérase, en tant qu'agent protecteur des mélanocytes du follicule pileux.. En particulier, l'agent mimétique de l'activité de la DOPAchrome tautomérase est destiné à lutter contre la disparition des mélanocytes du follicule pileux en maintenant et/ou régénérant la population des mélanocytes actifs du bulbe et des mélanocytes quiescents de la région supérieure du follicule pileux.

Le follicule pileux est une invagination tubulaire de l'épiderme qui s'enfonce jusqu'aux couches profondes du derme. La partie inférieure, ou bulbe pileux, comporte elle-même une invagination dans laquelle se trouve la papille dermique. La partie intérieure au bulbe est une zone de prolifération cellulaire où se trouvent les précurseurs des cellules kératinisées constituant le cheveu. Les cellules en ascension issues de ces précurseurs se kératinisent progressivement dans la partie supérieure du bulbe, et cet ensemble de cellules kératinisées formera la tige pilaire.

La couleur des cheveux et des poils repose notamment sur la présence en quantités et ratios variables de deux groupes de mélanines : les eumélanines (pigments bruns et noirs) et les phéomélanines (pigments rouges et jaunes). La pigmentation du cheveu et des poils requiert la présence de mélanocytes au niveau du bulbe du follicule pileux. Ces mélanocytes sont dans un état actif, c'est-à-dire qu'ils synthétisent des mélanines. Ces pigments sont transmis aux kératinocytes destinés à former la tige pilaire ce qui conduira à la pousse d'un cheveu ou d'un poil pigmenté. Cette structure est appelée ci-après « unité folliculaire de pigmentation ».
Chez les mammifères, la mélanogénèse implique au moins trois enzymes : la tyrosinase, la DOPAchrome tautomérase (TRP-2, pour Tyrosinase Related Protein 2) et la DHICAoxydase (TRP-1, pour Tyrosinase Related Protein 1).
La tyrosinase est l'enzyme qui initie la biosynthèse des mélanines. Elle est également décrite comme étant l'enzyme limitant de la mélanogénèse.
La TRP-2 catalyse la tautomérisation du DOPAchrome en acide 5,6-Dihydroxyindole-2-carboxylique (DHICA). En l'absence de TRP-2, le DOPAchrome subit une décarboxylation spontanée pour former le 5,6-dihydroxyindole (DHI).
DHICA et DHI sont tous deux des précurseurs de pigments, TRP-1 oxyde les molécules de DHICA pour former des dérivés de quinones (Pawelek JM and Chakraborty AK. The enzymology of melanogenesis. In: Nordlund JJ, Boissy RE, Hearing VJ, King RA, Ortonne J-P. The Pigmentary System: Physiology and Pathophysiology. New York: Oxford university press; 1998. p. 391-400).
Les trois enzymes, tyrosinase, TRP-2 et TRP-1, apparaissent spécifiquement impliquées dans la mélanogénèse. De plus, l'activité de ces trois enzymes a été décrite comme nécessaire à l'activité maximale de biosynthèse des eumélanines.
L'expression de TRP-2 a été observée dans les poils de souris noires, à la fois dans les mélanocytes actifs du bulbe et dans les mélanocytes quiescents de la gaine épithéliale externe. De plus, il est connu que l'activité DOPAchrome tautomérase est augmentée pendant la phase anagène chez la souris noire. Cependant aucune corrélation claire n'a été établie entre l'expression de TRP-2 et l'intensité de la pigmentation (Sturm et al. 1995).
Par ailleurs, TRP-2 a également été décrite comme conférant aux mélanocytes qui l'expriment une résistance à des agents endommageant l'ADN tel que le cis-diamminedichloroplatinum(II) (Chu et al. 2000 et Pak et al. 2000). Ces résultats suggèrent que TRP-2 serait aussi impliquée dans une fonction indépendante de la mélanogènèse, l'enzyme pourrait jouer un rôle cytoprotecteur.

Le cheveu et le poil subissent un cycle. Ce cycle comprend une phase de croissance (phase anagène), une phase de dégénérescence (phase catagène) et une phase de repos (phase télogène) à la suite de laquelle une nouvelle phase anagène se développera. En raison de ce cycle pilaire, et contrairement à l'unité de pigmentation épidermique, l'unité folliculaire de pigmentation doit également être cycliquement renouvelée.

Ce processus a été récemment décrit chez l'homme (Commo S. et Bernard B., 2000, Pigment Cell Res. 13:253-259). Il a plus particulièrement été montré qu'au cours de la transition télogène-anagène, une partie des mélanocytes inactifs contenus dans la capsule télogène prolifère, se positionne autour de la papille dermique du bulbe naissant et commence à exprimer des enzymes nécessaires à la synthèse de mélanines : cette population de mélanocytes correspond aux mélanocytes actifs du bulbe . En parallèle, l'autre partie des mélanocytes reste inactive dans la région supérieure du follicule pileux : cette population de mélanocytes correspond aux mélanocytes quiescents de la région supérieure du follicule pileux.
Ces enzymes mélanogènes seront exprimées dans les mélanocytes du bulbe pendant toute la durée de la phase-anagène mais ne seront plus exprimées pendant les phases catagène et télogène. Le cycle normal des mélanocytes dans le follicule pileux humain requiert la présence de mélanocytes quiescents dans la région supérieure du follicule pileux, région autrement nommée « réservoir », qui seront cycliquement activés pour régénérer l'unité folliculaire de pigmentation. Ce mécanisme de renouvellement cellulaire participant au maintien de la pigmentation est spécifique de l'unité folliculaire de pigmentation ; on ne le retrouve pas dans l'unité épidermique de pigmentation.

Il est admis que la canitie (blanchissement naturel des cheveux) est associée à une diminution de mélanine dans la tige pilaire. La cause de cette diminution n'est pas à ce jour élucidée. Plusieurs hypothèses sont avancées, elle pourrait être liée à une diminution de l'activité mélanogène, par analogie au mécanisme de pigmentation de la peau, mais également à une altération du transfert des mélanines ou à une diminution du nombre de mélanocytes dans le bulbe (Tobin et Paus, 2001); et aucune démonstration en pigmentation cheveu n'a permis à ce jour de valider l'une ou l'autre de ces hypothèses.→p 3a

Or la Demanderesse vient de mettre en évidence deux résultats qui valident pour la première fois l'hypothèse selon laquelle la canitie serait liée à une diminution du nombre de mélanocytes actifs dans le bulbe et une diminution du nombre de mélanocytes quiescents dans la région supérieure du follicule pileux. Cette diminution et/ou disparition précoce des mélanocytes est spécifique au follicule pileux et n'affecte pas de façon visible l'épiderme.

Jusqu'à présent, on pensait en effet que des mélanocytes quiescents étaient présents dans les follicules pileux de cheveux blancs (Takada et al. 1992, Horikawa et al. 1996, Jenner et Randall 2000).
Or, la Demanderesse a constaté que la progression de la canitie est associée à une diminution du nombre de mélanocytes dans les bulbes pileux, qui, bien qu'en nombre restreint, synthétisent et transfèrent les mélanines. La Demanderesse a également observé de façon inattendue et surprenante que la population de mélanocytes quiescents de la région supérieure du follicule pileux humain (encore appelée « réservoir ») est également diminuée au cours du processus de canitie, les cheveux blancs ne possédant plus que quelques - voire plus aucun - mélanocytes, contrairement à l'infundibulum et l'épiderme avoisinant ces cheveux blancs. Cette disparition affecte prématurément et spécifiquement les mélanocytes contenus dans les cheveux.

La demande internationale WO 99/39728 décrit une méthode mettant en oeuvre des neutrophines, leurs fragments, ou certains pseudo-ligands des facteurs de la croissance des nerfs, pour inhiber la mort cellulaire des mélanocytes de l'épiderme, mais pas des mélanocytes de follicule pileux.

La demande européenne EP 0 545 147 décrit l'utilisation de protéines conjuguées à des métaux, pouvant être par exemple la superoxyde dismutase, des glutathion-peroxydases ou un cytochrome c, pour empêcher ou diminuer le grisonnement des cheveux.

Le brevet américain US 5 965 157 décrit des formulations d'actifs pigmentants, dans des licosomes, pour favoriser leur adressage vers les follicules pileux.

La demande européenne EP 0 327 345 décrit l'utilisation du propyl gallate pour foncer les cheveux.

La demande européenne EP 0 580 409 décrit des acides aminés, dont la méthionine, et **des** dérivés N-acétylés d'acides aminés, comme agents stimulant la croissance **des** cheveux.

La demande espagnole ES 2 052 450 décrit un procédé d'obtention d'une composition comprenant **des** extraits végétaux pigmentants, utilisable comme lotion capillaire, ladite composition finale comprenant au moins de la méthionine à hauteur de 40 mg.

La demande japonaise JP 07 002677 décrit l'utilisation par voie orale de quercétine comme agent restaurant **les** cheveux, visant notamment la prévention de la canitie.

La demande japonaise JP 04 124122 décrit l'utilisation de quercétine comme composant d'un agent pour lutter contre **le** grisonnement **des** cheveux.

Le brevet américain US 5 696 109 décrit l'activité anti-oxydante de complexes salen-manganèse et leurs utilisations Pharmaceutiques et cosmétiques, n'incluant pas une utilisation comme protecteurs **des** mélanocytes ou agents actifs contre la dépigmentation **des** cheveux.

Il apparaît donc nécessaire de lutter contre la disparition des mélanocytes des follicules pileux humains, processus affectant à la fois les mélanocytes actifs des bulbes et les mélanocytes quiescents de la région supérieure des follicules pileux, pour lutter contre la canitie.

Par ailleurs, la Demanderesse a également constaté de façon inattendue que l'enzyme TRP-2 n'est pas exprimée dans les mélanocytes des follicules pileux humains pigmentés (bruns, noirs et roux) chez l'individu caucasien, asiatique et africain. Cette enzyme n'est détectée ni dans les mélanocytes actifs du bulbe, ni dans les mélanocytes quiescents de la région supérieure du follicule pileux humain alors qu'elle est exprimée dans l'épiderme et l'infundibulum de l'individu caucasien, africain et asiatique. L'absence de TRP-2 est associée à la disparition précoce des mélanocytes qui ne l'expriment pas, c'est-à-dire, les mélanocytes quiescents de la région supérieure du follicule pileux et les mélanocytes actifs du bulbe.
La Demanderesse a donc mis en évidence que TRP2, qui joue un rôle dans la mélanogénèse (synthèse de mélanine) au niveau de l'unité épidermique de pigmentation, pourrait jouer un rôle différent et méconnu jusqu'ici, dans l'unité folliculaire de pigmentation : la présence d'un composé à activité mimétique de TRP2 pourrait permettre de maintenir et/ou régénérer la population de mélanocytes quiescents de la région supérieure du follicule pileux et la population de mélanocytes actifs du bulbe et favoriser ainsi le renouvellement cyclique de l'unité folliculaire garant du maintien de la pigmentation des cheveux, cils et/ou poils.

La Demanderesse a identifié un moyen de maintenir et/ou régénérer la population de mélanocytes du follicule pileux responsables de la pigmentation des cheveux : en effet, elle a montré qu'il était possible de mimer l'activité TRP-2. Par ailleurs, elle a évalué l'activité cytoprotectrice d'agents mimétiques de TRP2 dans des conditions induisant l'apoptose et/ou la sénescence des mélanocytes du follicule pileux.
Elle a ainsi mis au point un moyen pour prévenir et/ou limiter et/ou stopper le développement de la canitie et même de maintenir la pigmentation naturelle des cheveux et/ou des poils gris ou blancs.

Ainsi présente demande décrit l'utilisation cosmétique d'un agent mimétique de l'activité de la DOPAchrome tautomérase, en tant qu'agent protecteur des mélanocytes du follicule pileux. →p4a

La Présente invention conceme en particulier l'utilisation cosmétique d'un agent mimétique de l'activité de la DOPAchrome tautomérase, en tant qu'agent protecteur des mélanocytes du follicule pileux, ledit agent mimétique de l'activité de la DOPAchrome tautomérase étant un complexe salen-manganèse.

Par 'agent protecteur des mélanocytes du follicule pileux', on entend un agent capable de protéger les mélanocytes du follicule pileux notamment contre des agents cytotoxiques responsables de la sénescence et/ou de l'apoptose des mélanocytes du follicule pileux. Parmi les agents cytotoxiques, on peut citer des molécules à caractères génotoxlques et des molécules induisant un stress oxydatif comme le TNF alpha, les lipofuscines, le TGF bêta, le ligand de Fas/CD95, L'IL1 beta, les ions ferreux et cuivreux, des composés chimiques génotoxiques comme le cisplatine et l'oxaloplatine, ou encore des composés comme le cyclophosphamide.

On entend par agent mimétique de l'activité de la DOPAchrome tautomérase un composé capable de reproduire les effets de la DOPAchrome tautomérase sur le métabolisme et la survie des mélanocytes.

En particulier, l'agent mimétique de l'activité de la DOPAchrome tautomérase selon l'Invention est destiné à lutter contre la disparition des mélanocytes du follicule pileux en maintenant et/ou en régénérant la population des mélanocytes actifs du bulbe et des mélanocytes quiescents de la région supérieure du follicule pileux.

L'agent mimétique de l'activité de la DOPAchrome tautomérase selon l'invention est également destiné à favoriser le renouvellement cyclique de l'unité folliculaire de pigmentation.

En particulier, la présente demande décrit l'utilisation d'un agent mimétique de l'activité de la DOPAchrome tautomérase pour prévenir et/ou limiter et/ou arrêter le développement de la canitie.

La Présente demande décrit aussi à l'utilisation d'un agent mimétique de l'activité de la DOPAchrome tautomérase pour maintenir la pigmentation naturelle des cheveux et/ou des poils gris.
En Particulier, l'objet de l'invention concerne l'utilisation d'un agent mimétique de l'activité de la DOPAchrome tautomérase pour prévenir et/ou limiter et/ou arrêter le développement de la canitie, ledit agent mimétique de l'activité de la DOPAchrome tautomérase étant un complexe salen-manganèse.
L'objet de l'invention se rapporte aussi à l'utilisation d'un agent mimétique de l'activité de la DOPAchrome tautomérase pour maintenir la pigmentation naturelle des cheveux et/ou des poils gris, ledit agent mimétique de l'activité de la DOPAchrome tautomérase étant un complexe salen-manaanèse.

La demande décrit des L'agents mimétiques de l'activité de la DOPAchrome tautomerase (TRP-2) pouvant être notamment choisi parmi les composés suivant :
- molécules synthétiques mimétiques de SOD (Métaphore), par exemples, les complexes de manganèse tels que décrits dans US 5637578, 5610293, 5874421 ;
- composés anti-oxydants : tels que des dérivés de tetrahydro-5,6,7,8-naphtaneol-1, en particulier ceux décrits dans le brevet EP 0 404 640, des dérivés benzohétérocyles oxygénés (voir le brevet EP 0 685 473), des dérivés de cyclodextrines (voir le brevet EP 0 778 287), des composés silicés dérivés d'acide ascorbique (voir le brevet WO 01/30784), de la pyrrolidone carboxylate de lysine ou d'arginine (voir le brevet EP 0 511 118), des dérivés de benzylidène camphre ter-butyle (voir le brevet US 4,952,391), des dérivés de benzylidène cyclanone (voir le brevet FR 2636531), des diorganopolysiloxanes modifiés (voir le brevet EP 0 370 868), des dérivés lipophiles du benzylidène camphre (voir le brevet EP 0 390 681), des dérivés hydrophile du benzylidène camphre (voir le brevet EP 0 390 682), des dérivés de benzyl-cyclanone (voir le brevet EP 0 390 683), des polymères anti-oxydants tels que décrits dans le brevet US 4,281,192, des associations de mono- et diester d'acide cinnamique et de vitamine C (voir le brevet EP 0 664 290), des polyamines, telles que putrescine, spermidine, spermine, Di-amines, Tri-amines, Tetra-amines ; le propyl gallate, la quercetine, le trolox, l'histidine, le tryptophane, la méthionine, des chélateurs de métaux (21-aminostéroides), le complexe salen-manganèse (par exemple : EUK-8), l'α-phenyl-ter-butyl nitrol (PBN) (et composés dérivés) ou l'ebselen ;
- composés non anti-oxydants, tel que le MIF et ses analogues (Macrophage Migration Inhibiting Factor, voir Rosengren et al. Mol. Med., 1996, 2(1 ):143-9).

La demande décrit aussi une composition pour lutter contre la canitie, comprenant dans un milieu cosmétiquement acceptable, au moins un agent mimétique de l'activité de la DOPA tautomérase (TRP2) tel que défini précédemment.

Un autre objet de l'invention est une composition pour lutter contre la canitie. comprenant dans un milieu cosmétiquement acceptable, au moins un complexe salen-manganèse, ledit complexe salen-manganèse étant associé à un autre actif choisi parmi les agents de lutte des états desquamatifs du cuir chevelu, des agents favorisant la repousse des cheveux, des extraits végétaux à activité propigmentante.

La composition selon l'invention comprend une quantité d'agent mimétique de l'activité de la DOPAchrome tautomérase comprise entre 0.001 % et 10 % en poids par volume, préférentiellement entre 0,01 et 5% en poids par volume et encore plus préférentiellement entre 0,1 et 1 % en poids par volume..

La composition selon l'invention peut être administrée par voie orale ou appliquée sur la peau (sur toute zone cutanée du corps recouverte de poils) et/ou le cuir chevelu.

Par voie orale, la composition selon l'invention peut contenir, le ou les agents mimétiques de l'activité de la DOPAchrome tautomérase composés actifs en solution dans un liquide alimentaire tel qu'une solution aqueuse ou hydroalcoolique, éventuellement aromatisée. Ils peuvent également être incorporés dans un excipient solide ingérable et se présenter par exemple sous forme de granulés, de pilules, de comprimés ou de dragées. Ils peuvent également être placés en solution dans un liquide alimentaire conditionné lui-même éventuellement dans des capsules ingérables.

Selon le mode d'administration, la composition de l'invention peut se présenter sous toutes les formes galéniques normalement utilisées, particulièrement en cosmétologie. Une composition préférée de l'invention est une composition cosmétique adaptée à une application topique sur le cuir chevelu et/ou la peau.

Pour une application topique, la composition utilisable selon l'invention peut être notamment sous la forme d'une solution aqueuse, hydroalcoolique ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Elle peut ainsi se présenter sous forme d'onguent, de teinture, de crème, de pommade, de poudre, de timbre, de tampon imbibé, de solution, d'émulsion.ou de dispersion vésiculaire, de lotion, de gel, de spray, de suspension, de shampooing, d'aérosol ou de mousse. Elles peuvent être anhydres ou aqueuses. Elle peut également consister en des préparations solides constituant des savons ou des pains de nettoyage.
Ces compositions sont préparées selon les méthodes usuelles.

La composition utilisable selon l'invention peut en particulier être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, de masque.

La composition cosmétique selon l'invention sera préférentiellement une crème, une lotion capillaire, un shampooing ou un après-shampooing.

Les quantités des différents constituants des compositions utilisables selon l'invention sont celles classiquement utilisées dans les domaines considérés.

Lorsque la composition utilisable selon l'invention est une émulsion, la proportion de la phase grasse peut aller de 5% à 80% en poids, et de préférence de 5% à 50% en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3% à 30% eh poids, et de préférence de 0,5 à 20% en poids par rapport au poids total de la composition. L'émulsion peut, en outre, contenir des vésicules lipidiques.

Lorsque la composition utilisable selon l'invention est une solution ou un gel huileux, la phase grasse peut représenter plus de 90% du poids total de la composition.

Dans uns variante de l'invention, la composition sera telle que l'agent mimétique de l'activité de la DOPAchrome tautomérase est encapsulé dans un enrobage tel que des microsphères, des nanosphères, des oléosomes ou des nanocapsules, l'enrobage sera choisi selon la nature chimique de l'agent mimétique de l'activité de la DOPAchrome tautomérase.

A titre d'exemple, les microsphères pourront être préparées selon la méthode décrite dans la demande de brevet EP 0 375 520.

Les nanosphères pourront se présenter sous forme de suspension aqueuse et être préparées selon les méthodes décrites dans les demandes de brevet FR 0015686 et FR 0101438.

Les oléosomes consistent en une émulsion huile dans eau formée par des globules huileux pourvus d'un enrobage cristal liquide lamellaire dispersé dans une phase aqueuse (voir les demandes de brevet EP 0 641 557 et EP 0 705 593).

L'agent mimétique de l'activité de la DOPAchrome tautomérase pourra aussi être encapsulé dans des nanocapsules consistant en un enrobage lamellaire obtenu à partir d'un tensio-actif siliconé (voir la demande de brevet EP 0 780115), les nanocapsules pourront également être préparées à base de polyesters sulfoniques hydrodispersibles (voir la demande de brevet FR 0113337).

L'agent mimétique de l'activité de la DOPAchrome tautomérase pourra également être complexé à la surface de globules huileux cationiques, quelques soit leur taille (voir les demandes de brevet EP 1 010 413, EP 1 010 414, EP 1 010 415, EP 1 010 416, EP 1 013 338, EP 1 016 453, EP 1 018 363, EP 1 020 219, EP 1 025 898, EP 1 120 101, EP 1 120 102, EP 1 129 684, EP 1 160 005 et EP 1 172 077).

L'agent mimétique de l'activité de la DOPAchrome tautomérase peut enfin être complexé à la surface de nanocapsules ou nanoparticules pourvues d'un enrobage lamellaire (Voir EP 0 447 318 et EP 0 557 489) et contenant un tensio-actif cationique à la surface (voir les références citées précédemment pour les tensio-actifs cationiques).

En particulier, on préférera une composition telle que l'enrobage dans lequel l'agent.. mimétique de l'activité de la DOPAchrome tautomerase a un diamètre inférieur ou égale à 10 µm. Lorsque l'enrobage ne forme pas une vésicule sphérique, on entend par diamètre la dimension la plus grande de la vésicule.

De façon connue, la composition selon l'invention peut contenir également des adjuvants habituels dans le domaine cosmétique, tels que les gélifiants hydrophiles ou lipophile, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01% à 10% du poids total de la composition. Ces adjuvants, selon leur nature, peuvent être introduits dans la phase grasse, dans la phase aqueuse et/ou dans les sphérules lipidiques.
Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{®} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

les compositions décrites dans la demande peuvent associer au moins un agent mimétique de l'activité de TRP-2 à d'autres agents actifs. Parmi ces agents actifs, on peut citer à titre d'exemple :
- les agents modulant la différenciation et/ou la prolifération et/ou la pigmentation des cellules de la peau tels que le rétinol et ses esters, la vitamine D et ses dérivés, les oestrogènes tels que l'oestradiol, les modulateurs de l'AMPc tels que les dérivés de POMC, l'adénosine, ou la forskoline et ses dérivés, les prostaglandines et leurs dérivés, la triiodotrionine et ses dérivés ;
- des extraits de végétaux tels que ceux d'Iridacées ou de soja, extraits pouvant alors contenir ou non des isoflavones ;
- des extraits de micro-organismes;
- les agents anti-radicaux libres tels que l'α-tocophérol ou ses esters, les superoxyde dismutases ou ses mimétiques, certains chélatants de métaux ou l'acide ascorbique et ses esters ;
- les anti-séborrhéiques tels que certains acides aminés soufrés, l'acide 13-cis rétinoïque, l'acétate de cyprotérone ;
- les autres agents de lutte contre les états desquamatifs du cuir chevelu comme le zinc pyrithione, le disulfure de sélénium, le climbazole, l'acide undécylénique, le Kétoconazole, la piroctone olamine (octopirox) ou la ciclopiroctone (ciclopirox) ;
   en particulier, il pourra s'agir d'actifs stimulant la repousse et/ou favorisant le ralentissement de la chute des cheveux, on peut plus particulièrement citer à titre non limitatif :
   - les esters d'acide nicotinique, dont notamment le nicotinate de tocophérol, le nicotinate de benzyle et les nicotinates d'alkyles en C₁-C₆ comme les nicotinates de méthyle ou d'hexyle ;
   - les dérivés de pyrimidine, comme le 2,4-diamino 6-piperidinopyrimidine 3-oxyde ou "Minoxidil" décrit dans les brevets US 4,139,619 et US 4,596,812 ; l'Aminexil ou 2,4 diamino pyrimidine 3 oxyde décrit dans WO96/09048 ;
   - les agents inhibiteur de la lipoxygenase ou inducteur de la cyclooxydase favorisant la repousse des cheveux comme ceux décrits par la Demanderesse dans la demande de brevet européen EP 0 648 488 ;
   - les agents antibactériens tels que les macrolides, les pyranosides et les tétracyclines, et notamment l'Erythromyclne ;
   - les agents antagonistes de calcium, comme la Cinnarizine, la Nimodipine et la Nifedipine ;
   - des hormones, telles que l'estriol ou des analogues, ou la thyroxine et ses sels ;
   - des agents antiandrogènes, tels que l'oxendolone, la spironolactone, le diéthylstilbestrol et la flutamide ;
   - des inhibiteurs stéroïdiens ou non stéroïdiens des 5-α-réductases tels que ceux décrits par la Demanderesse dans les demandes de brevet européen EP 0 964 852 et EP 1 068 858 ou encore le finastéride ;
   - des agonistes des canaux potassiques dépendant de l'ATP tels que la cromakalim et le nicorandil ;
   - des extraits végétaux à activité pro-pigmentante comme les extraits de chrysanthème tels que décrits dans FR 2768343 et les extraits de Sanguisorba décrits FR 2782920A1.

De préférence, l'agent mimétique de l'activité de la DOPAchrome tautomérase est associé à un autre actif choisi parmi les agents de lutte des états desquamatifs du cuir chevelu, des agents favorisant la repousse des cheveux, des extraits végétaux à activité propigmentante.

La présente demande décrit aussi un procédé de traitement cosmétique de la canitie caractérisé en ce qu'on administre ou qu'on applique sur la zone à traiter une composition telle que définie précédemment comprenant au moins un agent mimétique de l'activité de la DOPAchrome tautomérase.

La demande décrit aussi à-un procédé de traitement cosmétique destiné à maintenir la pigmentation naturelle des cheveux et/ou des poils gris ou blancs caractérisé en ce qu'on administre ou qu'on applique sur la zone à traiter une composition telle que définie précédemment comprenant au moins un agent mimétique de l'activité de la DOPAchrome tautomérase.

Un autre objet de la présente invention se rapporte à un procédé de traitement cosmétique non-thérapeutique de la canitie caractérisé en ce qu'on administre ou qu'on applique sur la zone à traiter une composition cosmétique comprenant, dans un milieu cosmétiquement acceptable, au moins un complexe salen-manganèse ou une composition selon l'invention telle que définie précédemment comprenant, dans un milieu cosmétiquement acceptable, au moins un complexe salen-manganèse, ledit complexe salen-manganése étant associé à un autre actif choisi parmi les agents de lutte des états desquamatifs du cuir chevelu, des agents favorisant la repousse des cheveux, des extraits végétaux à activité propiamentante.

L'invention se rapporte aussi à un procédé de traitement cosmétique destiné à maintenir la pigmentation naturelle des cheveux et/ou des poils gris ou blancs caractérisé en ce qu'on administre ou qu'on applique sur la zone à traiter une composition cosmétique comprenant, dans un milieu cosmétiquement acceptable, au moins un complexe salen-manganèse ou une composition selon l'invention telle que définie précédemment comprenant, dans un milieu cosmétiquement acceptable, au moins un complexe salen-manganèse, ledit complexe salen-manganèse étant associé à un autre actif choisi parmi les agents de lutte des états desquamatifs du cuir chevelu, des agents favorisant la repousse des cheveux, des extraits végétaux à activité propigmentante.

Les procédés de traitement de la canitie et de pigmentation des cheveux et/ou des poils gris ou blancs peuvent également consister en l'ingestion d'une composition comprenant au moins un agent mimétique de l'activité de la DOPAchrome tautomérase.

Les zones à traiter peuvent être, par exemple et sans aucune limitation, le cuir chevelu, les sourcils, la moustache et/ou la barbe et toute zone de la peau recouverte de poils.

Plus particulièrement, les procédés de traitement cosmétique de la canitie et de pigmentation naturelle des cheveux et/ou poils gris ou blancs décrits dans la demande consistent à appliquer une composition comprenant au moins un agent mimétique de l'activité de la DOPAchrome tautomerase choisi parmi :
- molécules synthétiques mimétiques de SOD (Métaphore), par exemples, les complexes de manganèse tels que décrits dans US 5637578, 5610293, 5874421 ;
- composés anti-oxydants : tels que des dérivés de tetrahydro-5,6,7,8-naphtaneol-1, en particulier ceux décrits dans le brevet EP 0 404 640, des dérivés benzohétérocyles oxygénés (voir le brevet EP 0 685 473), des dérivés de cyclodextrines (voir le brevet EP 0 778 287), des composés silicés dérivés d'acide ascorbique (voir le brevet WO 01/30784), de la pyrrolidone carboxylate de lysine ou d'arginine (voir le brevet EP 0 511 118), des dérivés de benzylidène camphre ter-butyle (voir le brevet US 4,952,391), des dérivés de benzylidène cyclanone (voir le brevet FR 2636531), des diorganopolysiloxanes modifiés (voir le brevet EP 0 370 868), des dérivés lipophiles du benzylidène camphre (voir le brevet EP 0 390 681), des dérivés hydrophile du benzylidène camphre (voir le brevet EP 0 390 682), des dérivés de benzyl-cyclanone (voir le brevet EP 0 390 683), des polymères anti-oxydants tels que décrits dans le brevet US 4,281,192, des associations de mono- et diester d'acide cinnamique et de vitamine C (voir le brevet EP 0 664 290), des polyamines, telles que putrescine, spermidine, spermine, Di-amines, Tri-amines, Tetra-amines ; le propyl gallate, la quercetine, le trolox, l'histidine, le tryptophane, la méthionine, des chélateurs de métaux (21-aminostéroïdes), le complexe salen-manganèse (par exemple : EUK-8), l'α-phenyl-*ter*-butyl nitrol (PBN) (et composés dérivés) ou l'ebselen ;
- composés non anti-oxydants, tel que le MIF et ses analogues (Macrophage Migration Inhibiting Factor, voir Rosengren et al. Mol. Med., 1996, 2(1):143-9).

Les procédés de traitement cosmétique pour lutter contre la canitie et/ou pour maintenir la pigmentation naturelle des cheveux et/ou des poils gris ou blancs peut par exemple consister à appliquer la composition sur les cheveux et le cuir chevelu, le soir, garder la composition toute la nuit et éventuellement effectuer un shampooing le matin ou laver les cheveux à l'aide de cette composition et à laisser à nouveau en contact quelques minutes avant de rincer. La composition conforme à l'invention s'est révélée particulièrement intéressante lorsqu'elle est appliquée sous forme de lotion capillaire, éventuellement rincée ou même sous forme d'un shampooing.

La présente demande décrit aussi une méthode d'identification d'un agent mimétique de l'activité de la DOPAchrome tautomérase consistant à déterminer la capacité d'un composé à diminuer l'induction d'une mort cellulaire dans des mélanocytes n'exprimant pas la DOPAchrome tautomerase en référence à une population de mélanocytes qui expriment la DOPAchrome tautomerase, la méthode comprend les étapes suivantes :
a- mise en culture d'une population de mélanocytes ;
b- séparation en deux populations de la population de mélanocytes obtenue à l'issue de l'étape (a), appelées respectivement Mel-A et Mel-B, mise en culture de Mel-A dans un milieu A où les mélanocytes expriment peu ou pas la DOPAchrome tautomérase et de Mel-B dans un milieu B contenant au moins un facteur favorisant l'expression de la DOPAchrome tautomerase ;
c- exposition de la population Mel-A à une condition induisant l'apoptose ou la sénescence en culture en présence (population Mel-A(+)) ou en l'absence (population Mel-A(-)) d'un composé pour lequel on souhaite tester l'activité mimétique de la DOPAchrome tautomerase ;
d- exposition de la population Mel-B à une condition induisant l'apoptose ou la sénescence en culture identique à la condition choisie à l'étape (c) en l'absence du composé pour lequel on souhaite tester l'activité DOPAchrome tautomerase ;
e- comparaison des réponses apoptotique ou sénescente de la population Mel-A(+) avec Mel-A(-) et de Mel-A(+) avec Mel-B ;
f- sélection des composés pour lesquels la réponse est telle que la cytotoxicité observée dans l'essai Mel-A(+) est moindre que celle observée dans l'essai Mel-A(-), et la cytotoxicité observée dans l'essai Mel-A(+) est proche de celle observée dans l'essai Mel-B.

dans un mode de réalisation particulier décrit ici, les mélanocytes sont mis en culture à l'étape (a) dans du milieu M2 (PromoCell, Heidelberg, D). La mise en culture de l'étape (a) peut être réalisée selon différentes modalités :
- dans un système de culture monocouche où les cellules sont ensemencées à même le plastique ;
- les cellules peuvent aussi être ensemencées sur un ou plusieurs composés de matrice extra-cellulaire, tels que collagène, élastine, fibronectine, laminine ;
- les cellules peuvent être cultivées dans un système tridimensionnel, tel qu'un cheveu disséqué, un cheveu arraché, une peau reconstruite in vitro.
Cette étape (a) est mise en oeuvre de préférence pendant un temps compris entre 2 et 18 heures nécessaire à l'adhérence des cellules.

Pour la réalisation des étapes suivantes, le milieu de culture de l'étape (a) est ensuite remplacé par un milieu approprié au test :
- pour l'étape (b), il s'agit :
   pour la population de mélanocytes Mel-A, d'un milieu ne favorisant pas l'expression de TRP-2 ou bien d'un milieu contenant un facteur réprimant l'expression de la DOPAchrome tautomerase pour la population Mel-A, ce facteur pouvant être un facteur neutralisant les ARN messagers codant pour la TRP2 (méthode anti-sens, siRNA) à l'aide de séquences nucléotidiques appropriées.
   pour la population de mélanocytes Mel-B d'un milieu favorisant l'expression de TRP-2 ; ce milieu peut également contenir un facteur induisant l'expression de la DOPAchrome tautomerase. Par exemple, ce facteur peut être choisi parmi : l'Hexamethylène Bisacetamide (HMBA), la glycyrrhizine, diethylstilbestrol, estradiol, kaempférol, forskoline.

L'expression de TRP2 peut aussi être obtenue après transfection de la région codante du gène humain de TRP-2 (par exemple tel que décrit dans genebank n°S69231, n° NM_001922, n°AJ000503) dans n'importe quel vecteur approprié (exemple : pcDNA^{©} Vector, invitrogen, Groningen, CH, N). Dans ce cas le test peut-être réalisé sur mélanocytes ou sur tout autre type de cellules de mammifères (exemple : fibroblastes, kératinocytes).

Selon un mode de réalisation particulier, on utilisera à l'étape (b), pour la population de mélanocytes Mel-A, un milieu contenant un facteur neutralisant les ARN messagers codant pour la TRP2 (méthode anti-sens, siRNA) à l'aide de séquences nucléotidiques appropriées.

Comme oligonucléotide double brin capable de provoquer l'extinction de l'ARN messager codant pour la TRP2, on pourra utiliser les 3 séquences nucléotidiques suivantes (positions 838, 1589 et 2164 respectivement du gène codant pour TRP2) :

Les SEQ ID de numéro impair correspondent au brin 5' antisens ; les SEQ ID de numéro pair correspondent au brin 5' sens.
838
   SEQ ID N°1 : 5'- AACATCCATTCCTTGAGTCCTCCTGTCTC -3'
   SEQ ID N°2 : 5'- AAAGGACTCAAGGAATGGATGCCTGTCTC -3'
1589
   SEQ ID N°3: 5'- AAGTGATGAGCCTTCATAATTCCTGTCTC -3'
   SEQ ID N°4: 5'- AAAATTATGAAGGCTCATCACCCTGTCTC -3'
2164
   SEQ ID N°5: 5'- AATCCTCACTGTTCCTTCTTGCCTGTCTC -3'
   SEQ ID N°6: 5'- AACAAGAAGGAACAGTGAGGACCTGTCTC -3'

La fonctionnalité de ces séquences SiRNAs, comme inhibiteurs spécifiques de la TRP2, est vérifiée par une mesure en Western blot de l'extinction de l'expression de la TRP2 dans des mélanocytes en présence de ces SiRNAs sous forme de duplex (brin antisens et brin sens).

Pour la réalisation des étapes (c) et (d), les populations Mel-A et Mel-B sont exposées à une condition induisant l'apoptose ou la sénescence en culture, il pourra s'agir, par exemple, d'un facteur pro-apoptotique (TNF α), de l'absence d'un facteur de survie (IGF-1), d'un traitement par le cis-platine (Pak B.J. et al., 2000, Melanoma Res. 10 :499-505) ou l'oxaliplatine, d'un agent toxique (cyclophosphamide), d'un stress oxydatif (H₂O₂, diethylmaleate) (voir Vaux D.L. & Strasser A., 1996, Proc.Natl.Acad.Sci. 93 :2239-2244).

Pour la réalisation de l'étape (e), on pourra utiliser les méthodes de révélation de l'apoptose ou de sénescence suivantes :
- la réponse apoptotique peut être déterminée par toute méthode permettant de révéler l'apoptose cellulaire, par exemple, identification de la fragmentation de l'ADN après électrophorèse sur gel d'agarose, marquage des fragments d'ADN par la méthode du "TUNEL" (Gavrieli Y et al. J Cell Biol 1992 ;119 :493-501), révélation de l'anexine V (ApoAlert Annexin V Apoptosis Kit (1996) CLONTECHniques XI(3) :9-11 (BD Biosciences, Belgium mesure de l'activité de caspase (ApoAlert Caspase Assay Kit (BD Biosciences, Belgium). En particulier, l'apoptose peut être quantifiée à l'aide du kit « Cell Death Détection ELISA plus, mis en oeuvre selon le protocole donné par le fournisseur (Roche 1 774 425).
- la réponse sénescente peut être déterminée par toute méthode permettant de révéler la sénescence cellulaire, par exemple, détermination d'un raccourcissement des télomères, mesure de l'activité de la télomérase (TRAPeze kit, Intergen), détermination de la diminution du taux de cycline E, détermination de la diminution du taux de protéine P105 Rb phosphorylée (Bandyopadhyay D et al. Experimental Gerontology 2001 ;36 :1265-1275), mesure de l'activité beta-Galactosidase (Dimri GP et al. PNAS 1995 ;92 :9363-9367)

La demande décrit aussi l'utilisation d'un agent mimétique de l'activité de la DOPAchrome tautomérase susceptible d'être identifié par la méthode décrite ci-dessus dans un procédé de traitement cosmétique pour prévenir et/ou limiter et/ou arrêter le développement de la canitie et/ou pour maintenir la pigmentation naturelle des cheveux et/ou des poils gris ou blancs.

La demande décrit enfin l'utilisation d'un agent mimétique de l'activité de la DOPAchrome tautomerase susceptible d'être identifié par la méthode décrite ci-dessus pour la préparation d'une composition cosmétique destinée à prévenir et/ou limiter et/ou arrêter le développement de la canitie et/ou à maintenir la pigmentation naturelle des cheveux et/ou des poils gris ou blancs.

La demande décrit encore une méthode d'évaluation de l'activité cytoprotectrice d'un agent mimétique de l'activité de la DOPAchrome tautomerase susceptible d'être identifié par la méthode décrite précédemment, comprenant les étapes suivantes :
a- mise en culture d'une population de mélanocytes dans un milieu limitant l'expression de la TRP-2 à une expression basale faible ;
b- ajout d'un composé mimétique de l'activité de la DOPAchrome tautomérase au milieu de culture ;
c- exposition des cellules à une condition induisant l'apoptose ou la sénescence ;
d- mesure de la cytotoxicité ;
e- sélection des composés mimant l'activité de la DOPAchrome tautomérase avec un effet cytoprotecteur.

Dans un mode de réalisation particulier, les cultures cellulaires sont réalisées en étuve, à 37°C, 5% CO₂.
En particulier, l'étape (a) pourra être réalisée selon le protocole suivant : les mélanocytes sont ensemencés à J0 avec du milieu M2 (PromoCell, Heidelberg, D). Après un temps nécessaire à l'adhérence des cellules compris entre 2 et 18 heures, le milieu est remplacé par un milieu dans lequel les mélanocytes expriment peu ou pas la TRP-2 (expression faible basale) :DMEM :F12 (Gibco BRL - 42400-044), Ultroser G (Gibco BRL - 15950-017) 0,5%, PC-1 (BioWhittaker 344022) 0,5%, bFGF (Pepro Tech Inc 100-18B) 5 ng/ml, héparine (Sigma H-3149) 75 ng/ml, 1% antibiotiques, 1% glutamine. Les cellules sont maintenues dans ce milieu de culture le temps compris entre 12 et 72 heures nécessaire à la diminution de l'expression de TRP-2.

L'étape (b) pourra être réalisée selon le protocole suivant : les mélanocytes sont traités en culture par le composé mimétique de l'activité de la DOPAchrome tautomérase.

L'étape (c) pourra être réalisée par exemple selon le protocole suivant : les cellules sont traitées au cis-platine (par exemple entre 5 et 50 µM) dans le milieu de culture pendant le temps nécessaire à l'induction de l'apoptose, ce temps est généralement compris entre 12 et 24 heures.

L'étape (d) pourra être réalisée par exemple selon le protocole suivant : la cytotoxicité peut être mesurée à l'aide du kit « Cell Proliferation Kit II (XTT) » mis en oeuvre selon le protocole donné par le fournisseur (Roche 1-465-015). L'apoptose peut être quantifiée à l'aide du kit « Cell Death Detection ELISA plus, mis en oeuvre selon le protocole donné par le fournisseur (Roche 1 774 425).

### Figures :

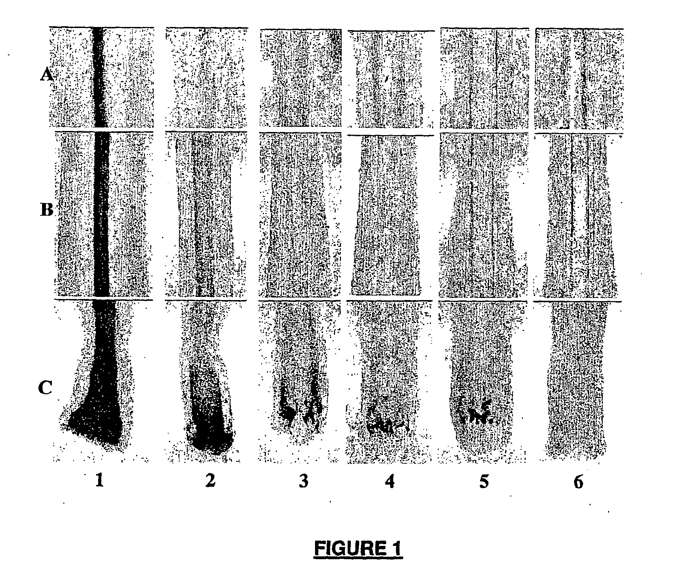

**Figure 1** : cette figure rassemble différentes photographies représentant la distribution de mélanocytes dans le follicule pileux lors de la phase anagène visualisée au microscope.
   Légendes :
   (A) est une série de clichés de la gaine épithéliale externe grossie 40 fois, (B) est une série de clichés de la gaine épithéliale externe (centrés sur la tige) grossie 20 fois et (C) est une série de clichés du bulbe grossie 20 fois. (1) représente un cheveu très foncé, (2) un cheveu modérément pigmenté, (3) à (5) des cheveux de différentes nuances de gris et (6) un cheveu blanc.
**Figure 2** : Ces photographies permettent de visualiser l'expression de TRP-2 dans les mélanocytes de l'épiderme et du cheveu (gaine épithéliale externe et bulbe pileux). Etude immunohistologique analysée en microscopie confocale laser.
**Figure 3** : Ces photographies représentent les résultats obtenus après la mise en oeuvre des essais de Western Blot décrits à l'exemple 2B.

**Exemple 1** - **révélation immunohistochimique des mélanocytes dans** les **follicules pileux à différents stades de blanchissement, par marguage de la protéine pMel-17**.

Plus de 120 follicules pileux isolés à partir de biopsies issues de 8 donneurs âgés de 49 à 71 ans ont été étudiés.
A - Protocole d'isolement des follicules pileux entiers (Commo S and Bernard BA Pigment Cell Res 2000 ;13 :253-259)
   Des fragments de biopsie sont incubés dans la dispase (2,4 U/ml, Boehringer Mannheim, D) la nuit à +04°C. Les cheveux sont ensuite isolés à l'aide de pinces sous binoculaires.
B - Protocole d'immunomarquage sur follicules pileux entiers (Commo S and Bernard BA Pigment Cell Res 2000 ;13 :253-259)

Les cheveux entiers sont fixés dans l'éthanol à -20°C pendant 10 minutes. Chaque étape des procédures de fixation et de marquage est suivie de lavages au tampon phosphate (pH 7,4 (PBS))-Tween20 0,05%. Sauf précision, toutes les étapes se font à température ambiante. Les péroxydases endogènes de l'échantillon sont neutralisées en incubant l'échantillon dans une solution de péroxyde d'hydrogène à 0,1% pendant 10 minutes. Pour bloquer les sites de fixation non spécifiques, l'échantillon est incubé avec du lait écrémé 1%, 15 minutes. L'anticorps (Ac) primaire NK1-beteb reconnaissant spécifiquement la protéine pMel-17 (Monosan, Paris, F) est dilué au 1/40 dans du PBS - Tween 0,05%, contenant 10 % de sérum normal (X0907, DAKO, Trappes, F). L'Ac primaire est incubé 18 heures sur les cheveux à +04°C. L'Ac secondaire couplé à la biotine (E-433, DAKO, Trappes, F) est dilué au 1/400 et incubé 30 minutes. Le cheveu est ensuite incubé en présence de streptavidine-biotine-péroxydase (K-0377, DAKO, Trappes, F), et finalement l'immunomarquage est révélé en présence de 3-amino-9-éthylcarbazole (AEC) (AEC kit-101, Sigma, Saint Quentin Fallavier, F).

En comparant les clichés (B1) à (B5) de la figure 1, on constate que la diminution de pigmentation du cheveu est associé à une diminution de mélanine dans le bulbe et à une diminution de mélanocytes dans le bulbe (voir C1 à C5). Le cheveu blanc dont la tige est dépourvue de mélanine (B6) ne contient pas de mélanocyte dans le bulbe (C6). Les cheveux gris et blanc contiennent une quantité variable de mélanocytes dans la partie haute de la gaine épithéliale externe, cette quantité pouvant même être nulle dans le cas du cheveu blanc (A3 à 6) à la différence des cheveux pigmentés (A1 et 2).

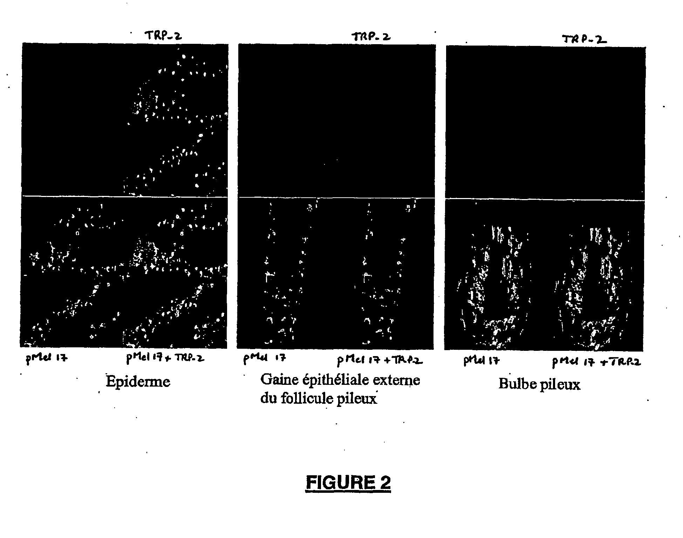

**Exemple 2 - mise en évidence de l'expression différentielle de la DOPAchrometautomérase dans les mélanocytes de follicules pileux et de l'épiderme chez l'individu caucasien.**
A - Etude immunohistologique analysée en microscopie confocale laser
A.1 - Obtention de coupes congelées de follicule pileux (Commo S and Bernard BA Pigment Cell Res 2000 ;13 :253-259)
   Un fragment de biopsie de scalp contenant des follicules pileux est inclus dans du tissue-Tek-OCT (Miles, Naperville, IL, USA) et ensuite congelé sur de la carbo-glace. La biopsie congelée est ensuite coupée (7 µm) à l'aide d'une cryostat (CM3050, Leica, Rueil-Malmaison, F).
A.2 - Protocole d'isolement des follicules pileux entiers et des lambeaux épithéliaux de peau (Commo S and Bernard BA Pigment Cell Res 2000 ;13 :253-259)
Des fragments de biopsie sont incubés dans la dispase (2,4 U/ml, Boehringer Mannheim, D) la nuit à +04°C. Le compartiment épithélial est séparé du derme à l'aide de pinces sous binoculaire. Les structures épithéliales sont ensuite micro-disséquées pour séparer les follicules pileux et l'épiderme, puis triées.
A.3 - Protocole d'immunomarquage sur follicules pileux entiers, lambeau de peau et coupe congelée
   Les cheveux entiers, les lambeaux épithéliaux de peau et les coupes congelées sont fixés dans l'éthanol à -20°C pendant 10 minutes. Chaque étape des procédures de fixation et de marquage est suivie de lavages au tampon phosphate (pH 7,4 (PBS))-Tween20 0,05%. Sauf précision, toutes les étapes se font à température ambiante: Les péroxydases endogènes de l'échantillon sont neutralisées en incubant l'échantillon dans une solution de péroxyde d'hydrogène à 0,1% pendant 10 minutes. Pour bloquer les sites de fixation non spécifiques, l'échantillon est incubé avec du lait écrémé 1%, 15 minutes. Les anticorps (Ac) primaires sont dilués dans du PBS - Tween 0,05%, contenant 10 % de sérum normal (X0907, DAKO, Trappes, F). Les Ac primaires NK1-beteb reconnaissant spécifiquement la protéine pMel-17 (1/40, Monosan, Paris, F), et αPEP8h (1/2000, Dr VJ.Hearing, NIH, Bethesda, MD, USA) reconnaissant spécifiquement la protéine TRP2 humaine (Virador et al. 2001) sont incubés simultanément 18 heures à +04°C sur les cheveux entiers et les lambeaux épithéliaux de peau, et 30 minutes à température ambiante sur les coupes congelées. L'Ac secondaire de chèvre dirigé contre les immunoglobulines (Ig) G2b couplé à la Cy3 (M32410, TEBU, le Perray en Yveline, F) est dilué au 1/80, et l'Ac secondaire dirigé contre les Ig couplé à la Cy5 (111-175-144, Jackson Immunoresearch Lab. Inc. West Grove, PA, USA) est dilué au 1/500 sont incubés simultanément 30 minutes sur les échantillons. Les immunomarquages sont analysés en microscopie confocale laser (LSM510, Carl Zeiss, Oberkochen, D).
   Conclusion des observations : on constate sur la figure 2 la présence de TRP-2 dans les mélanocytes de l'épiderme, en revanche, cette enzyme n'est exprimée ni dans les mélanocytes de la gaine épithéliale du follicule pileux, ni dans les mélanocytes du bulbe pileux.
B - étude biochimique par analyse en Western Blot
B.1 - Protocole d'extraction de protéine de follicules pileux humains et de mélanocytes (Commo S et al. Differentiation 2000 ;66 :157-164)

- Extraction protéique à partir de bulbes pileux : les follicules pileux sont isolés après traitement de biopsies de scalp à la dispase (2,4 U/ml, Boehringer Mannheim, D) la nuit à +04°C. Après isolement, les follicules pileux sont micro-disséqués pour isoler la partie du bulbe pileux. 80 bulbes pileux ainsi isolés sont placés dans un tampon de lyse approprié pour extraction protéique et analyse en *western blot.*
- Extraction protéique à partir de culture de mélanocytes : Les mélanocytes cultivés en milieu M2 (PromoCell, Heidelberg, D) sont lysés avec un même tampon de lyse approprié pour extraction protéique et analyse en *western blot.*

Le *Western blot* (voir protocole dans Maniatis *et al.*) est réalisé avec les anticorps suivants : αPEP8h, anticorps polyclonal spécifique de la TRP-2 humaine donné par Dr VJ Hearing (NIH, Bethesda, USA), et T311, anticorps monoclonal spécifique de la tyrosinase humaine (Novocastra, New Castle, UK).

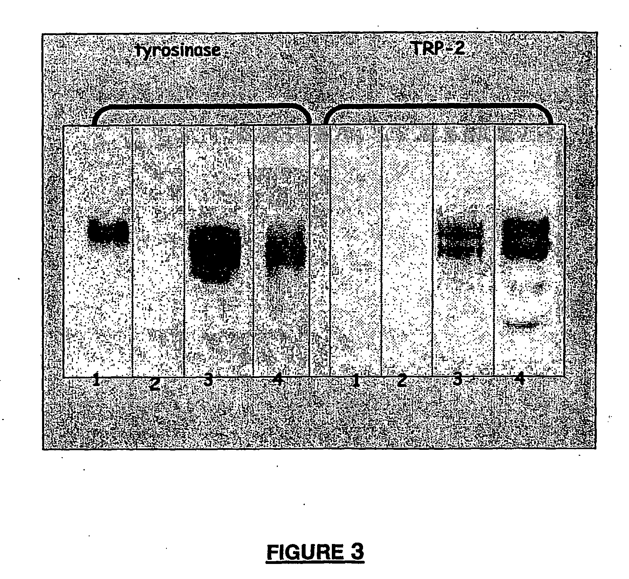

On observe (figure 3) que la tyrosinase est détectée dans les extraits de bulbe pileux. L'enzyme n'est pas détectée dans les extraits de gaine épithéliale externe. L'expression de la tyrosinase est régulée. Cette enzyme n'est pas ou peu exprimée dans les mélanocytes inactifs (ne produisant pas de mélanine), c'est le cas des mélanocytes contenus dans le scalp inter folliculaire d'individu caucasien.
Par ailleurs, la DOPAchrome tautomérase (TRP-2) n'est détectée ni dans les extraits de bulbe ni dans les extraits de gaine épithéliale externe. L'expression de la TRP-2 ne suit pas celle de la tyrosinase et l'induction de la mélanogénèse, elle n'est pas exprimée dans les mélanocytes actifs des bulbes pileux.

### Exemple 3 - Compositions

### - lotion capillaire

| | | |
|---|---|---|
| Agent mimétique de l'activité de la DOPAchrome tautomérase | | 0,5 g |
| Propylène glycol | | 20 g |
| Ethanol 95° qsp | | 30 g |

Cette lotion est appliquée quotidiennement sur les zones à traiter et de préférence sur l'ensemble du cuir chevelu pendant au moins 10 jours et préférentiellement 1 à 2 mois. On constate alors une diminution de l'apparition des cheveux blancs ou gris et une repigmentation des cheveux gris.

### - shampooing traitant -

| | |
|---|---|
| Agent mimétique de l'activité | |
| de la DOPAchrome tautomérase | 1,5 g |
| Polyglycéryl 3-hydroxylarylether | 26 g |
| Hydroxy propyl cellulose vendue sous la dénomination de Klucell G par la société Hercules | 2 g |
| Conservateurs | ps |
| Ethanol 95° | 50 g |
| Eau qsp | 100 g |

Ce shampooing est utilisé à chaque lavage avec un temps de pose d'environ d'une minute. Un usage prolongé, de l'ordre de deux mois, conduit à la repigmentation progressive des cheveux gris.
Ce shampooing peut également être utilisé à titre préventif afin de retardé le blanchiment des cheveux.

### - Gel traitant

| | |
|---|---|
| Agent mimétique de l'activité de la DOPAchrome tautomérase | 0,75 g |
| Huiles essentielles d'Eucalyptus | 1 g |
| Econozole | 0,2 g |
| Lauryl polyglyceryl 6 cetearyl glycoether | 1,9 g |
| Conservateurs | qs |
| Carbopol 934P vendu par la société BF Goodrich Corporation | 0,3 g |
| Agent de neutralisation | qs pH 7 |
| Eau qsp | 100 g |

Ce gel est appliqué sur les zones à traiter deux fois par jour (matin et soir) avec un massage terminal. Après trois mois d'application, on observe une repigmentation des poils ou cheveux de la zone traitée.

### LISTE DES SEQUENCES

SEQ ID N°1: 5'- AACATCCATTCCTTGAGTCCTCCTGTCTC -3'
SEQ ID N°2: 5'- AAAGGACTCAAGGAATGGATGCCTGTCTC -3'
SEQ ID N°3: 5'- AAGTGATGAGCCTTCATAATTCCTGTCTC-3'
SEQ ID N°4: 5'- AAAATTATGAAGGCTCATCACCCTGTCTC -3'
SEQ ID N°5: 5'- AATCCTCACTGTTCCTTCTTGCCTGTCTC -3'
SEQ ID N°6: 5'-AACAAGAAGGAACAGTGAGGACCTGTCTC -3'

## Revendications

1. Utilisation cosmétique non thérapeutique un agent mimétique de l'activité de la DOPAchrome tautomérase, en tant qu'agent protecteur des mélanocytes du follicule pileux, ledit agent mimétique de l'activité de la DOPAchrome tautomérase étant un complexe salen-manganèse.

2. Utilisation selon la revendication 1, **caractérisée en ce que** l'agent mimétique de l'activité de la DOPAchrome tautomérase est destiné à lutter contre la disparition des mélanocytes du follicule pileux en maintenant et/ou en régénérant la population des mélanocytes actifs du bulbe et des mélanocytes quiescents de la région supérieure du follicule pileux.

3. Utilisation selon l'une des revendications 1 ou 2, **caractérisée en ce que** l'agent mimétique de l'activité de la DOPAchrome tautomérase est destiné à favoriser le renouvellement cyclique de l'unité folliculaire de pigmentation.

4. Utilisation selon l'une des revendications 1 à 3, **caractérisée en ce que** l'agent mimétique de l'activité de la DOPAchrome tautomérase est destiné à prévenir et/ou limiter et/ou arrêter le développement de la canitie.

5. Utilisation selon l'une des revendications 1 à 3, **caractérisée en ce que** l'agent mimétique de l'activité de la DOPAchrome tautomérase est destiné à maintenir la pigmentation naturelle des cheveux et/ou des poils gris.

6. Composition cosmétique pour lutter contre la canitie comprenant, dans un milieu cosmétiquement acceptable, au moins un complexe salen-manganèse, ledit complexe salen-manganèse étant associé à un autre actif choisi parmi les agents de lutte des états desquamatifs du cuir chevelu, des agents favorisant la repousse des cheveux, des extraits végétaux à activité propigmentante.

7. Composition selon la revendication 6, **caractérisée en ce qu'**elle comprend une quantité d'agent mimétique de l'activité de la DOPAchrome tautomérase comprise entre 0,001 et 10 % poids par volume, préférentiellement entre 0,01 et 5% en poids par volume et encore plus préférentiellement entre 0,1 et 1 % en poids par volume.

8. Composition selon l'une quelconque des revendications 6 à 7, **caractérisée en ce qu'**elle est adaptée à une administration par voie orale.

9. Composition selon l'une quelconque des revendications 6 à 7, **caractérisée en ce qu'**elle est adaptée à une application topique sur le cuir chevelu et/ou sur les zones de la peau recouvertes de poils.

10. Composition selon la revendication 9, **caractérisée en ce qu'**elle est une crème ou un gel coiffant, une lotion capillaire, en particulier une lotion de mise en plis ou une lotion traitante ou une lotion restructurante pour les cheveux, une composition de teinture, un shampoing ou un après-shampoing.

11. Composition selon l'une quelconque des revendications 6 à 10, **caractérisée en ce que** l'agent mimétique de l'activité de la DOPAchrome tautomérase est encapsulé dans un enrobage tel que des microsphères, des nanosphères, des oléosomes ou des nanocapsules.

12. Composition selon la revendication 11, **caractérisée en ce que** l'enrobage dans lequel l'agent mimétique de l'activité de la DOPAchrome tautomerase est encapsulé a un diamètre inférieur ou égale à 10 µm.

13. Procédé de traitement cosmétique non-thérapeutique de la canitie **caractérisé en ce qu'**on administre ou qu'on applique sur la zone à traiter une composition cosmétique comprenant, dans un milieu cosmétiquement acceptable, au moins un complexe salen-manganèse ou une composition selon l'une des revendications 6 à 12.

14. Procédé de traitement cosmétique non-thérapeutique destiné à maintenir la pigmentation naturelle des cheveux et/ou des poils gris ou blancs **caractérisé en ce qu'**on administre ou qu'on applique sur la zone à traiter une composition cosmétique comprenant, dans un milieu cosmétiquement acceptable, au moins un complexe salen-manganèse ou une composition selon l'une des revendications 6 à 12.

## Patentansprüche

1. Nicht-therapeutische kosmetische Verwendung eines Mimetikums der Aktivität der DOPAchrom-Tautomerase als Mittel zum Schutz der Melanozyten des Haarfollikels, wobei das Mimetikum der Aktivität der DOPAchrom-Tautomerase ein Salen-Mangan-Komplex ist.

2. Verwendung nach Anspruch 1, **dadurch gekennzeichnet, dass** das Mimetikum der Aktivität der DOPAchrom-Tautomerase dazu dient, den Verlust der Melanozyten des Haarfollikels zu bekämpfen, indem die Population der aktiven Melanozyten des Bulbus und der ruhenden Melanozyten der oberen Region des Haarfollikels erhalten und/oder regeneriert wird.

3. Verwendung nach einem der Ansprüche 1 oder 2, **dadurch gekennzeichnet, dass** das Mimetikum der Aktivität der DOPAchrom-Tautomerase dazu dient, die cyclische Erneuerung der Pigmentierungseinheit des Follikels zu fördern.

4. Verwendung nach einem der Ansprüche 1 bis 3, **dadurch gekennzeichnet, dass** das Mimetikum der Aktivität der DOPAchrom-Tautomerase dazu dient, die Entwicklung von Canities zu verhindern und/oder zu beschränken und/oder anzuhalten.

5. Verwendung nach einem der Ansprüche 1 bis 3, **dadurch gekennzeichnet, dass** das Mimetikum der Aktivität der DOPAchrom-Tautomerase dazu dient, die natürliche Pigmentierung grauer Haare und/oder grauer Körperbehaarung zu erhalten.

6. Kosmetische Zusammensetzung zur Bekämpfung von Canities, die in einem kosmetisch annehmbaren Medium mindestens einen Salen-Mangan-Komplex umfasst, wobei der Salen-Mangan-Komplex mit einem anderen Wirkstoff vereinigt ist, der aus Mitteln zur Bekämpfung desquamativer Zustände der Kopfhaut, Mitteln, die das Nachwachsen der Haare fördern, pflanzlichen Extrakten mit propigmentärer Aktivität ausgewählt ist.

7. Zusammensetzung nach Anspruch 6, **dadurch gekennzeichnet, dass** sie eine Menge des Mimetikums der Aktivität der DOPAchrom-Tautomerase zwischen 0,001 und 10 Vol.-%, vorzugsweise zwischen 0,01 und 5 Vol.-% und noch stärker bevorzugt zwischen 0,1 und 1 Vol.-% umfasst.

8. Zusammensetzung nach einem der Ansprüche 6 bis 7, **dadurch gekennzeichnet, dass** sie an eine Verabreichung auf oralem Weg angepasst ist.

9. Zusammensetzung nach einem der Ansprüche 6 bis 7, **dadurch gekennzeichnet, dass** sie an eine topische Anwendung auf die Kopfhaut und/oder auf die Bereiche der Haut, die von Körperhaaren bedeckt sind, angepasst ist.

10. Zusammensetzung nach Anspruch 9, **dadurch gekennzeichnet, dass** sie eine Frisiercreme oder ein Frisiergel, eine Haarlotion, insbesondere eine Wasserwellenlotion oder eine Behandlungslotion oder eine Umstrukturierungslotion für die Haare, eine Färbezusammensetzung, ein Shampoo oder eine Spülung ist.

11. Zusammensetzung nach einem der Ansprüche 6 bis 10, **dadurch gekennzeichnet, dass** das Mimetikum der Aktivität der DOPAchrom-Tautomerase in eine Umhüllung, wie Mikrosphären, Nanosphären, Oleosomen oder Nanokapseln, eingekapselt ist.

12. Zusammensetzung nach Anspruch 11, **dadurch gekennzeichnet, dass** die Umhüllung, in der das Mimetikum der Aktivität der DOPAchrom-Tautomerase eingekapselt ist, einen Durchmesser von unter oder gleich 10 µm hat.

13. Verfahren zur nicht-therapeutischen kosmetischen Behandlung von Canities, **dadurch gekennzeichnet, dass** man auf den zu behandelnden Bereich eine kosmetische Zusammensetzung verabreicht oder aufbringt, die in einem kosmetisch annehmbaren Medium mindestens einen Salen-Mangan-Komplex oder eine Zusammensetzung nach einem der Ansprüche 6 bis 12 umfasst.

14. Verfahren zur nicht-therapeutischen kosmetischen Behandlung, das dazu dient, die natürliche Pigmentierung grauer oder weißer Haare und/oder grauer oder weißer Körperbehaarung zu erhalten, **dadurch gekennzeichnet, dass** man auf den zu behandelnden Bereich eine kosmetische Zusammensetzung verabreicht oder aufbringt, die in einem kosmetisch annehmbaren Medium mindestens einen Salen-Mangan-Komplex oder eine Zusammensetzung nach einem der Ansprüche 6 bis 12 umfasst.

## Claims

1. Non-therapeutic cosmetic use of a DOPAchrome tautomerase activity mimetic, as a protective agent for hair follicle melanocytes, said DOPAchrome tautomerase activity mimetic being a salen-manganese complex.

2. Use according to Claim 1, **characterized in that** the DOPAchrome tautomerase activity mimetic is intended for combating the disappearance of hair follicle melanocytes by maintaining and/or regenerating the population of active melanocytes of the bulb and of quiescent melanocytes of the upper region of the hair follicle.

3. Use according to either of Claims 1 or 2, **characterized in that** the DOPAchrome tautomerase activity mimetic is intended for promoting the cyclic renewal of the follicular pigmentation unit.

4. Use according to one of Claims 1 to 3, **characterized in that** the DOPAchrome tautomerase activity mimetic is intended for preventing and/or limiting and/or stopping the development of canities.

5. Use according to one of Claims 1 to 3, **characterized in that** the DOPAchrome tautomerase activity mimetic is intended for maintaining the natural pigmentation of grey hair and/or grey body hair.

6. Cosmetic composition for combating canities comprising, in a cosmetically acceptable medium, at least one salen-manganese complex, said salen-manganese complex being combined with another active agent chosen from agents for combating desquamative conditions of the scalp, agents for promoting hair regrowth and plant extracts with propigmenting activity.

7. Composition according to Claim 6, **characterized in that** it comprises an amount of DOPAchrome tautomerase activity mimetic of between 0.001% and 10% weight by volume, preferentially between 0.01% and 5% weight by volume and even more preferentially between 0.1% and 1% weight by volume.

8. Composition according to any one of Claims 6 to 7, **characterized in that** it is suitable for oral administration.

9. Composition according to any one of Claims 6 to 7, **characterized in that** it is suitable for topical application to the scalp and/or to the areas of the skin covered with body hair.

10. Composition according to Claim 9, **characterized in that** it is a styling gel or cream, a hair lotion, in particular a hair setting lotion or a medicated lotion or a restructuring lotion for the hair, a dyeing composition, a shampoo or a hair conditioner.

11. Composition according to any one of Claims 6 to 10, **characterized in that** the DOPAchrome tautomerase activity mimetic is encapsulated in a coating such as microspheres, nanospheres, oleosomes or nanocapsules.

12. Composition according to Claim 11, **characterized in that** the coating in which the DOPAchrome tautomerase activity mimetic is encapsulated has a diameter of less than or equal to 10 µm.

13. Non-therapeutic cosmetic treatment process for canities, **characterized in that** a cosmetic composition comprising, in a cosmetically acceptable medium, at least one salen-manganese complex or a composition according to one of Claims 6 to 12 is administered or applied to the area to be treated.

14. Non-therapeutic cosmetic treatment process intended for maintaining the natural pigmentation of grey or white hair and/or body hair, **characterized in that** a cosmetic composition comprising, in a cosmetically acceptable medium, at least one salen-manganese complex or a composition according to one of Claims 6 to 12 is administered or applied to the area to be treated.
